# EUROPÄISCHE PATENTSCHRIFT

(11) **EP 3 369 803 B1**
(45) Veröffentlichungstag und Bekanntmachung des Hinweises auf die Patenterteilung: **28.08.2019**
(21) Anmeldenummer: 18152391.1
(22) Anmeldetag: 18.01.2018
(51) Int. Cl.: C12M 1/00, C12M 1/38, B01L 7/00, C12M 1/34, F24F 6/02, F24F 11/00

(54) **VERFAHREN ZUR BEFEUCHTUNG EINES INKUBATORS UND INKUBATOR**
INCUBATOR AND METHOD FOR HUMIDIFYING AN INCUBATOR
PROCÉDÉ D'HUMIDIFICATION D'UN INCUBATEUR ET INCUBATEUR

(30) Priorität: 03.03.2017 DE 102017104508
(43) Veröffentlichungstag der Anmeldung: 05.09.2018
(73) Patentinhaber: Adolf Kühner AG, 4127 Birsfelden (CH)
(72) Erfinder: Cesana, Christoph, 4059 Basel (CH); Batlle, Joan, 4052 Baselstadt (CH); Bürgin, Tim, 4410 Liestal (CH); Knobel, Simon, 4133 Pratteln (CH); Anderlei, Tibor, 79379 Müllheim (DE); Schumacher, Mathias, 79539 Lörrach (DE)
(74) Vertreter: Kohlmann, Kai

(56) Entgegenhaltungen:
- EP-A1- 2 573 162
- EP-A1- 2 963 105
- DE-A1-102013 009 136
- DE-C1- 3 630 886
- DE-U1-202007 005 865
- JP-A- H07 318 268

## Beschreibung

Die Erfindung betrifft ein Verfahren zur geregelten Befeuchtung und Temperierung eines Gasgemisches in einem gegen die Umgebung mittels eines Gehäuses abgedichteten Innenraum eines Inkubators.

Außerdem betrifft die Erfindung einen Inkubator mit geregelter Befeuchtung und Temperierung eines Gasgemisches in einem abgedichteten Innenraum.

Inkubatoren dienen dazu, in ihrem Innenraum Proben unter vorgegebenen Bedingungen, wie einer bestimmten Temperatur und Luftfeuchtigkeit sowie in einer definierten Gasatmosphäre zu lagern. Eine typische Innenraumtemperatur bewegt sich zwischen 20-40 Grad Celsius bei einer Feuchtigkeit des Gasgemisches in dem Innenraum im Bereich von 70 - 90%. Außerdem sind sogenannte Schüttelinkubatoren bekannt, die ideale Bedingungen für Kultivierungsprozesse bereit stellen. Die Feuchteregelung in Inkubatoren, insbesondere Schüttelinkubatoren ist wichtig, damit insbesondere bei lang andauernden Kultivierungen von bis zu 14 Tagen und sehr kleinen Kulturflüssigkeitsvolumina, insbesondere in Mikrotiterplatten, die Verdunstung reduziert wird. Eine hohe Verdunstung führt zu einer Änderung des Kultivierungsmediums und beeinflusst das Wachstum und den Stoffwechsel der Zellen. Eine Reduzierung der Verdunstung wird durch eine Befeuchtung des Gasgemisches im Innenraum des Inkubators erreicht. Dabei gilt der Grundsatz, dass je höher die Feuchtigkeit des Gasgemisches im Innenraum ist, desto geringer ist die Verdunstung des Kulturmediums. Bei sehr hohen Feuchtigkeitswerten von über 85% besteht jedoch die Gefahr, dass Feuchtigkeit an Innenflächen des Inkubators aufgrund nicht vollständig homogener Temperaturverteilung auskondensiert. Derartige sich in Folge der Auskondensierung bildende Feuchtigkeitsbereiche stellen jedoch potenzielle Kontaminationsherde dar.

Aus der DE 10 2006 022 652 B4 ist ein Schüttel-Inkubator und ein Verfahren zur Befeuchtung und Temperierung der Luft in dem Innenraum des Inkubators bekannt, bei dem der Luft aus einer Feuchtequelle einer Befeuchtungsvorrichtung Feuchtigkeit zugeführt und die Luft über eine Inkubatorheizung und/oder Inkubatorkühlung temperiert wird. Durch zwei voneinander getrennte Luftkreisläufe kann unter Verwendung einer herkömmlichen Befeuchtungsvorrichtung die Feuchtigkeit der Luft in dem Innenraum nahezu unabhängig von der Temperaturregelung mithilfe der Inkubatorheizung und/oder Kühlung geregelt werden. In bekannter Weise kommt ein temperaturgeregeltes Wasserbad als Befeuchtungsvorrichtung zum Einsatz. Die Feuchtigkeit in dem Innenraum wird mit einem Feuchtesensor gemessen. Die Regelung der Feuchte erfolgt durch Regelung des Luftstroms in dem ersten, die Befeuchtungsvorrichtung aufweisenden Luftkreislaufs und durch Regulierung des Heizelements des Wasserbades.

Mit dem bekannten Inkubator sowie dem Verfahren zur geregelten Befeuchtung und Temperierung der Luft in dem Innenraum des Inkubators ist es nicht möglich, die Feuchtigkeit aktiv zu reduzieren. Hierzu muss der Luft Feuchtigkeit entzogen werden.

Die Adolf Kühner AG Dinkelbergstr. 1, 4127 BIRSFELDEN (Basel), Schweiz stellt u.a. unter der Bezeichnung ISF1-X Schüttel-Inkubatoren mit einer optionalen Feuchtigkeitsregelung her. Zum Erhöhen der Feuchtigkeit des Gasgemisches ist im Innenraum des Inkubators ein Wasserbad angeordnet. Die Temperatur im Wasserbad wird mit einer elektrischen Heizung erhöht und steigert durch Verdampfen des Wassers die Feuchtigkeit des Gasgemisches im Innenraum. Um die Feuchtigkeit aktiv zu reduzieren, ist ein Kühlkreislauf einer Kompressor-Kühlung in den Inkubator integriert. Der Verdampfer des Kühlkreislaufs befindet sich als Kondensationsfläche im Innenraum. Das feuchte Gasgemisch kondensiert an dem Verdampfer, der so angebracht ist, dass das kondensierte Wasser zurück in das Wasserbad tropft bzw. fließt. Die Regelung erfolgt mit Hilfe eines kapazitiven Sensors zum Messen des Istwertes der Feuchtigkeit des Gasgemisches in dem Innenraum. Ein digitaler PID-Regler erhöht die Feuchtigkeit durch Verdampfen von Wasser aus dem elektrisch beheizten Wasserbad, sofern der Istwert aufgrund der kontinuierlichen Reduzierung der Feuchtigkeit an dem Verdampfer kleiner als der Sollwert ist. Der kontinuierliche Wärmeabfluss an dem Verdampfer wird durch Temperieren des Gasgemisches mit einer Heizung auf einen gewünschten Wert der Innenraumtemperatur nachgeführt.

Bei dem Inkubator ISF1-X der Firma Kühner AG übernimmt die Kompressor-Kühlung zwei Aufgaben. Zum einen kann sie die Innenraumtemperatur absenken und zum anderen die Feuchtigkeit des Gasgemisches im Innenraum reduzieren. Inkubatoren, insbesondere Schüttelinkubatoren, werden von deren Anwendern jedoch häufig bei einer konstanten Innenraumtemperatur in einem Bereich von 32 - 37 Grad Celsius betrieben, sodass eine Absenkung der Innenraumtemperatur nicht erforderlich ist. Das Kühlaggregat ist jedoch für die Feuchtigkeitsregelung erforderlich.

Die EP 2 573 162 A1 offenbart einen Inkubator mit geregelter Temperierung eines Gasgemisches in einem abgedichteten Innenraum, wobei das Gasgemisch beispielsweise eine Temperatur von 37 Grad Celsius aufweist. Der Inkubator weist ein wärmeisolierendes Gehäuse mit einer Öffnung an seiner Vorderseite auf, die mittels einer wärmeisolierenden Tür geöffnet und geschlossen werden kann. Auf dem Boden des Innenraums des Inkubators ist eine Befeuchtungsschale angeordnet, wobei die Befeuchtungsschale zum Speichern von Wasser vorgesehen ist, um die Feuchtigkeit im Inneren des Inkubators zu steuern. Der Inkubator umfasst darüber hinaus einen Wärmeleiter, der einen wärmeisolierenden Abschnitt, einen Wärmeeinleit-Abschnitt und einen Wärmeableit-Abschnitt umfasst. Der Wärmeleiter ist derart angeordnet, dass der Wärmeableit-Abschnitt außerhalb des Inkubators angeordnet ist, während der Wärmeeinleit-Abschnitt in der Leitung derart angeordnet ist, dass an dem Wärmeeinleit-Abschnitt kondensiertes Wasser nach unten fließt und in die Befeuchtungsschale eintritt. Sobald die Feuchtigkeit im Innenraum des Inkubators sich gesättigtem Wasserdampf annähert, kondensiert Feuchtigkeit an dem Wärmeinleit-Abschnitt des Wärmeleiters und das kondensierte Wasser fließt in die Befeuchtungsschale zurück und kann wiederholt als Befeuchtungswasser dienen. Um die Wärmeableitung über den Wärmeableit-Abschnitt zu verbessern, kann an dem Wärmeableit-Abschnitt eine Wärmesenke angebracht werden. Alternativ kann zur Unterdrückung einer zu starken Abnahme der Feuchtigkeit im Innenraum des Inkubators eine Heizung an den Wärmeableit-Abschnitt außerhalb des Inkubators angeschlossen werden.

Die DE 36 30 886 C1 betrifft eine Klimaprüfkammer mit einem Nutzraum und einem daran angrenzenden Luftbehandlungsraum. In dem Luftbehandlungsraum ist ein erster Wärmetauscher zur Temperierung der Nutzraumluft mit einem Umluftventilator angeordnet. Unterhalb des ersten Wärmetauschers befindet sich in dem Luftbehandlungsraum eine heizbare Wasserschale. Der Wasserschale ist eine Kühleinheit zugeordnet. Die Kühleinheit ist als zweiter Wärmetauscher, insbesondere als Rohrkühler, ausgeführt. Der zweite Wärmetauscher ist in unmittelbarer Nähe der Oberfläche des Wassers in der Wasserschale angeordnet. Zum Befeuchten der Luft wird die Heizung der heizbaren Wasserschale eingeschaltet, sodass Wasser aus der Wasserschale verdampft und der vorbeiströmenden Nutzraumluft zugeführt wird. Beim Entfeuchten wird flüssiges Kältemittel über ein Ringventil und ein Expansionsventil in den Rohrkühler eingespritzt, wodurch einerseits die Wasseroberflächentemperatur unmittelbar herabgesetzt wird und andererseits der vorbeiströmende Nutzraumluft Feuchtigkeit entzogen wird.

Ausgehend von diesem Stand der Technik liegt der Erfindung die Aufgabe zugrunde ein Verfahren zur geregelten Befeuchtung und Temperierung eines Gasgemisches in einem gegen die Umgebung mittels eines Gehäuses abgedichteten Innenraum eines Inkubators zu schaffen, das kein Kühlaggregat benötigt und zugleich Kontaminationsherde durch auskondensierende Feuchtigkeit vermeidet. Außerdem soll ein Inkubator zur Durchführung des Verfahrens angegeben werden.

Diese Aufgabe wird durch die Merkmale des unabhängigen Anspruchs 1 sowie des unabhängigen Anspruchs 11 gelöst.

Ein wesentlicher Gesichtspunkt der Erfindung besteht darin, Wärme von der mindestens einen Kondensations-Oberfläche innerhalb des Inkubators mittels mindestens eines Wärmeleiters durch eine den Innenraum begrenzende Wand aus dem Inkubator nach Außen herauszuleiten. Außerhalb des Inkubators ist jeder Wärmeleiter mit einem Kühlkörper verbunden, der die Wärme an die kältere Umgebungsluft abgibt.

Die erfindungsgemäße Lösung ist preiswerter als die bekannte Lösung mit einem Kühlaggregat. Hinzu kommt die Robustheit aufgrund der einfachen Ausführung. Durch die Anordnung des Kühlkörpers außerhalb des Gehäuses als separates Bauteil, kann die erfindungsgemäße Lösung an bestehenden Inkubatoren problemlos nachgerüstet werden.
Insbesondere können Kühlaggregate mit klimaschädlichen Kältemitteln auf Kundenwunsch ausgebaut und durch die erfindungsgemäße Lösung ersetzt werden.

Um den Wärmeübergang von dem Kühlkörper an die Umgebungsluft zu verbessern, kann die wirksame Oberfläche des Kühlkörpers profiliert sein. Beispielsweise kann der profilierte Kühlkörper mehrere, parallel zueinander angeordnete Lamellen aufweisen. Um den Wärmewiderstand des Kühlkörpers gering zu halten besteht dieser vorzugsweise aus gut wärmeleitendem Material, wie beispielsweise Kupfer oder Aluminium. Die Oberfläche des Kühlkörpers ist vorzugsweise mit einer korrosionsbeständigen, antihaftenden und abriebfesten Schicht versehen. Hierdurch wird ein Schutz der Oberfläche gegen Beschädigung, Korrosion und Umwelteinflüsse geschaffen. Der Kühlkörper ist als von dem Gehäuse separates Bauteil ausgeführt und vorzugsweise an einer der Seitenwände des Gehäuses des Inkubators befestigt.

Zur Anbindung des Kühlkörpers an den mindestens einen Wärmeleiter, weist dieser vorzugsweise ein plattenförmiges, massives Bauteil mit einer Öffnung zur formschlüssigen Aufnahme eines Endes jedes Wärmeleiters auf. Das plattenförmige Bauteil besteht ebenfalls aus einem gut wärmeleitenden Material, insbesondere aus Aluminium oder Kupfer. Das plattenförmige Bauteil ist vorzugsweise mit einer Wand des Gehäuses des Inkubators verschraubt. Zur Verschraubung können seitlich an dem plattenförmigen Bauteil ansetzende Flansche vorgesehen sein.

Zum Übertragen der Wärme von der Kondensations-Oberfläche zu dem Kühlkörper kommt mindestens ein Wärmeleiter zum Einsatz. Bei den Wärmeleitern handelt es sich beispielsweise um Stäbe aus gut wärmeleitendem Material, wie beispielsweise Aluminium oder Kupfer. In besonders bevorzugter Ausführung der Erfindung handelt es sich bei dem Wärmeleiter um ein Wärmerohr, auch als "Heatpipe" bezeichnet. Ein wesentlicher Vorteil des Wärmerohrs besteht darin, dass dessen Wärmewiderstand bei Arbeitstemperatur deutlich kleiner als der Wärmewiderstand von Metallen ist. Bei gleicher Wärmeübertragungsleistung ist daher bei Verwendung von Wärmerohren eine kompaktere Bauweise als bei herkömmlichen Wärmeleitern aus Metall möglich. Um während der Übertragung der Wärme von der Kondensations-Oberfläche zu dem außerhalb des Gehäuses angeordneten Kühlkörper Wärmeverluste und Kondensatbildung an dem im Innenraum verlaufenden Abschnitt des Wärmeleiters zu vermeiden, ist dieser in vorteilhafter Ausgestaltung der Erfindung mit einer Wärmedämmung versehen. In Längsrichtung erstreckt sich die Wärmedämmung vorzugsweise über die gesamte Länge des Wärmeleiters mit Ausnahme der Enden, über die die Wärme eingekoppelt bzw. auf den Kühlkörper übertragen wird. Insbesondere umgibt die Wärmedämmung jeden Wärmeleiter auch im Bereich der Durchtrittsöffnungen in der Gehäusewand, sodass lediglich die gewollte, durch den Wärmeleiter hergestellte Kältebrücke zu dem Kühlkörper gebildet wird. Die den Wärmeleiter umgebende Dämmung liegt bündig an den die Austrittsöffnungen begrenzenden Flächen an. Eine Auskühlung der Gehäusewand und dadurch bedingte ungewollte Kondensation an einer Innenfläche der Gehäusewand wird indes vermieden.

Das Temperieren des Gasgemisches auf eine im technischen Sinne konstante Innenraumtemperatur, typischerweise im Bereich zwischen 32-37 Grad Celsius (Kultivierungstemperatur) erfolgt mittels einer geregelten Heizung. Um eine gleichmäßige Temperaturverteilung im Innenraum zu gewährleisten, können die Wände des Inkubators einschließlich der verschließbaren Öffnung, insbesondere der Tür mit Wand-Heizelementen versehen sein. Hierdurch wird eine ungewollte Kontamination durch Kondensation von Feuchtigkeit an Teilbereichen der Wände und/oder der Tür vermieden.

Zur Befeuchtungsregelung weist der Inkubator eine Befeuchtungsvorrichtung auf, deren Verdampfungsleistung einstellbar ist. Als Befeuchtungsvorrichtung kommen grundsätzlich beheizbare Flüssigkeitsbäder, insbesondere Wasserbäder, Ultraschallverdampfer sowie beheizte Platten, auf die Flüssigkeit tropft, in Betracht. In bevorzugter Ausführungsform des Inkubators weist die Befeuchtungsvorrichtung einen im Innenraum angeordneten Behälter zur Aufnahme der zu verdampfenden Flüssigkeit auf. Der Behälter weist mindestens eine die Oberfläche der Flüssigkeit freigebende Öffnung auf. Vorzugsweise ist die gesamte Oberseite des Behälters offen, sodass dieser sich als Wanne beziehungsweise Schale darstellt. Ferner verfügt der Behälter über eine Heizung zur Zuführung thermischer Energie in die Flüssigkeit. Die Heizung umfasst eines oder mehrere an der Außenseite des Behälters oder in die Flüssigkeit eintauchende Heizelemente.

Bevorzugt wird das beheizbare Flüssigkeitsbad unmittelbar im Innenraum des Inkubators auf dessen Bodenfläche, nicht jedoch in etwaigen Gasführungskanälen des Innenraums angeordnet. Der Behälter zur Aufnahme des Flüssigkeitsbades ist durch diese Anordnung gut zugänglich und kann problemlos gereinigt und/oder entnommen und gereinigt werden. Kontaminationen durch verkeimte Flüssigkeit in dem Behälter lassen sich dadurch vermeiden. Insbesondere erlaubt diese offene Anordnung des Behälters eine vorteilhafte Anordnung der Kondensationsoberfläche(n).

Bei der ersten Ausführungsform der Erfindung ist es zweckmäßig, wenn die Wärmeleiter aus mehreren, lösbar miteinander verbundenen Bestandteilen bestehen. Zur Reinigung des Behälters können der Wärmeaustauschkörper und die in den Behälter ragenden Abschnitte jedes Wärmeleiters problemlos demontiert werden.

Zum Reduzieren der Feuchtigkeit des Gasgemisches durch Kondensation ist in dem Innenraum mindestens eine Kondensationsoberfläche angeordnet. Bei der Kondensationsoberfläche handelt es sich um eine definierte, von den Innenflächen des Gehäuses verschiedene Oberfläche. Durch die Abgrenzung der Kondensationsoberfläche von dem Gehäuse können unkontrollierte Kondensationen sowie Ansammlungen von kondensiertem Wasser in dem Gehäuse vermieden werden.

Eine Kontamination des Innenraums des Inkubators durch kondensierende Feuchtigkeit wird in einer ersten Ausführungsform der Erfindung besonders wirksam dadurch vermieden, dass die Feuchtigkeit des Gasgemisches an der Oberfläche der Flüssigkeit in dem Behälter kondensiert, die zugleich die Kondensationsoberfläche bildet. Die Wärme wird von der Flüssigkeitsoberfläche mithilfe des mindestens einen Wärmeleiters zu dem außerhalb des Gehäuses angeordneten Kühlkörpers übertragen. Das von dem Kühlkörper abgewandte Ende jedes Wärmeleiters taucht zu diesem Zweck in die Flüssigkeit ein. Um den Wärmeübergang zu verbessern, ist das Ende des mindestens einen Wärmeleiters mit einem Wärmeaustauschkörper wärmeleitend verbunden, wobei der Wärmetauschkörper zumindest teilweise, vorzugsweise jedoch vollständig in die Flüssigkeit eintaucht. Der Wärmetauschkörper besteht ebenfalls aus einem gut wärmeleitenden Material, wie beispielsweise Kupfer oder Aluminium.

Vorzugsweise ist der Wärmetauschkörper so nah am Boden des Behälters angeordnet, dass dieser bei Erreichen des tiefsten Flüssigkeitsspiegels in dem Behälter noch vollständig mit Flüssigkeit bedeckt ist. Jeder Wärmeleiter kann ausgehend von dem Wärmetauschkörper über die Wasseroberfläche aus dem Behälter durch die die Oberfläche freigebende Öffnung herausgeführt werden. Alternativ kann der Wärmeleiter durch eine Wand des Behälters herausgeführt werden.

In einer zweiten Ausführungsform der Erfindung kondensiert die Feuchtigkeit an der Oberfläche eines von dem Inkubatorgehäuse separaten Wärmetauschkörpers, der im Innenraum des Inkubators angeordnet ist. Die Oberfläche des Wärmetauschkörpers bildet dabei die Kondensations-Oberfläche. Der Wärmetauschkörper besteht aus gut wärmeleitendem Material, wie insbesondere Kupfer oder Aluminium. Mit dem Wärmetauschkörper ist der mindestens eine Wärmeleiter zum Übertragen der Wärme von dem Wärmetauschkörper zu dem außerhalb des Gehäuses angeordneten Kühlkörper verbunden.

Zur Vergrößerung der durch den Wärmetauschkörper gebildeten Kondensationsoberfläche kann dieser strukturiert sein, beispielsweise durch parallel zueinander angeordnete Lamellen. Zur Vermeidung von Kontaminationen des Innenraums durch an dem Wärmetauschkörper kondensierende Feuchtigkeit ist der Wärmetauschkörper derart oberhalb der mindestens einen Öffnung in dem Behälter angeordnet, dass das Kondensat unmittelbar in den darunter angeordneten Flüssigkeitsbehälter fließt bzw. abtropft.

Bei beiden Ausführungsformen des erfindungsgemäßen Inkubators wird der Wärmeübergang von dem Kühlkörper zu der Umgebungsluft durch Verändern der Strömung der Umgebungsluft entlang der wärmeabgebenden Oberflächen des Kühlkörpers mittels mindestens eines in der Drehzahl steuerbaren Lüfters verändert. Bei dem Lüfter handelt es sich beispielsweise um einen Axialventilator dessen Drehachse im Wesentlichen senkrecht zu der Wand steht, an der der Kühlkörper befestigt ist. Der Lüfter wird zweckmäßigerweise an dem Kühlkörper befestigt, sodass der Kühlkörper und der mindestens eine Lüfter eine Baueinheit bilden.

Der Lüfter kann in die Feuchtigkeitsregelung eingebunden werden, sodass der Luftdurchsatz durch den Kühlkörper bedarfsweise reduziert werden kann. Durch eine Reduktion des Luftdurchsatzes lässt sich die kontinuierliche Übertragung von Wärme zu dem Kühlkörper reduzieren, sodass bei hohen Sollwerten für die Feuchtigkeit des Gasgemisches eine geringere Verdampferleistung erforderlich ist. Insbesondere können zum Erreichen hoher Sollwerte der Feuchtigkeit des Gasgemisches der oder die Lüfter abgeschaltet oder in ihrer Drehzahl reduziert werden.

Um eine gleichmäßige Temperatur- und Feuchtigkeitsverteilung im Innenraum des Inkubators zu gewährleisten, wird das Gasgemisch im Innenraum kontinuierlich umgewälzt. Die Umwälzung erfolgt insbesondere mittels mindestens eines im Innenraum angeordneten Lüfters. Der Lüfter ist beispielsweise als Axialventilator ausgestaltet, der an der Decke des Inkubators befestigt ist.

Die Feuchtigkeitsregelung des Inkubators umfasst mindestens einen im Innenraum des Gehäuses angeordneten Feuchtigkeitssensor zur Messung der Feuchtigkeit des Gasgemisches im Innenraum. Dabei handelt es sich insbesondere um einen kapazitiven Sensor dessen elektrische Kapazität sich mit der Feuchtigkeit ändert. Der Feuchtigkeitssensor misst die tatsächliche Feuchtigkeit, d.h. den Istwert des Gasgemisches in dem Gehäuse.

Die Feuchtigkeitsregelung regelt die Feuchtigkeit des Gasgemisches in dem Innenraum des Inkubators auf einen einstellbaren, im Wesentlichen konstanten Sollwert. Die beispielsweise als PID-Regler ausgeführte Feuchtigkeitsregelung vergleicht den Istwert mit dem Sollwert für die Feuchtigkeit.

Sofern eine Abweichung des Istwertes von dem Sollwert vorliegt, wird in der ersten Ausführungsform der Erfindung die Verdampfungsleistung mit der Heizung derart geregelt, dass
- keine Feuchtigkeit an der Flüssigkeitsoberfläche kondensiert und so viel Flüssigkeit verdampft, bis der Sollwert der Feuchtigkeit erreicht ist, wenn der gemessene Istwert kleiner als der Sollwert ist. Die Zufuhr von thermischer Energie zu der Flüssigkeit in dem Behälter überwiegt die kontinuierliche Ausleitung von Energie mittels des mindestens einen Wärmeleiters zu dem Kühlkörper, so dass Flüssigkeit aus dem Behälter verdampfen kann oder
- Feuchtigkeit an der Flüssigkeitsoberfläche kondensiert und keine Flüssigkeit verdampft, bis der Sollwert der Feuchtigkeit erreicht ist, wenn der gemessene Istwert größer als der Sollwert ist. Die Zufuhr von thermischer Energie zu der Flüssigkeit in dem Behälter wird soweit gedrosselt oder vollständig unterbunden, so dass die kontinuierliche Ausleitung von Energie mittels des mindestens einen Wärmeleiters aus dem Flüssigkeitsbad zu dem Kühlkörper zu einer so starken Abkühlung der Flüssigkeitsoberfläche führt, dass die Feuchtigkeit an der Flüssigkeitsoberfläche kondensiert.

Sofern eine Abweichung des Istwertes von dem Sollwert vorliegt, wird in der zweiten Ausführungsform der Erfindung die Verdampfungsleistung mit der Heizung derart geregelt, dass
- so viel Flüssigkeit im Verhältnis zu der kontinuierlich an der Kondensationsoberfläche kondensierenden Feuchtigkeit verdampft, bis der Sollwert der Feuchtigkeit erreicht ist, wenn der gemessene Istwert kleiner als der Sollwert ist. Die Zufuhr von thermischer Energie zu der Flüssigkeit in dem Behälter bewirkt, dass mehr Flüssigkeit verdampft als an dem Wärmeaustauschkörper kondensiert, oder
- keine oder so wenig Flüssigkeit im Verhältnis zu der kontinuierlich an der Kondensationsoberfläche kondensierenden Feuchtigkeit verdampft, bis der Sollwert der Feuchtigkeit erreicht ist, wenn der gemessene Istwert größer als der Sollwert ist. Die Drosselung oder Unterbrechung der Zufuhr thermischer Energie zu der Flüssigkeit in dem Behälter bewirkt, dass keine oder weniger Flüssigkeit verdampft als an dem Wärmeaustauschkörper kondensiert.

Ein Inkubator mit automatischer Füllstandskontrolle des Flüssigkeitsbehälters zur Aufnahme der zu verdampfenden Flüssigkeit zeichnet sich dadurch aus, dass in dem Behälter ein Füllstandssensor für die Flüssigkeit angeordnet ist, in dem Behälter eine Flüssigkeitszufuhr mündet, die mit einer Flüssigkeitsversorgung verbunden ist und eine Regelung für die Füllstandskontrolle derart eingerichtet ist, dass bei Unterschreiten eines von dem Füllstandssensor erfassten unteren Grenzwert des Füllstands, die Flüssigkeit in dem Behälter über die Flüssigkeitszufuhr automatisch aufgefüllt wird.

Der erfindungsgemäße Inkubator kann als Brutschrank oder als Schüttelinkubator verwendet werden. Für den Einsatz als Schüttelinkubator weist der Inkubator zusätzlich eine in dem Innenraum angeordnete Schütteleinrichtung für Gefäße mit zu kultivierenden Medien auf. Die Schütteleinrichtung ist insbesondere zur Aufnahme von Mikrotiterplatten zur Aufnahme der zu kultivierenden Medien eingerichtet. Die gewünschte Reduzierung der Verdunstung bei biotechnologischen Prozessen wird durch die erfindungsgemäße Feuchtigkeitsregelung erreicht, die die Feuchtigkeit auf einem zuvor gewählten Sollwert konstant hält.

Nachfolgend wird die Erfindung anhand der Ausführungsbeispiele näher erläutert. Es zeigen
- **Figur 1**: ein erstes Ausführungsbeispiel eines erfindungsgemäßen Inkubators,
- **Figur 2**: ein Abwandlung des ersten Ausführungsbeispiels des erfindungsgemäßen Inkubators nach Figur 1 sowie
- **Figur 3**: ein zweites Ausführungsbeispiel des erfindungsgemäßen Inkubators.

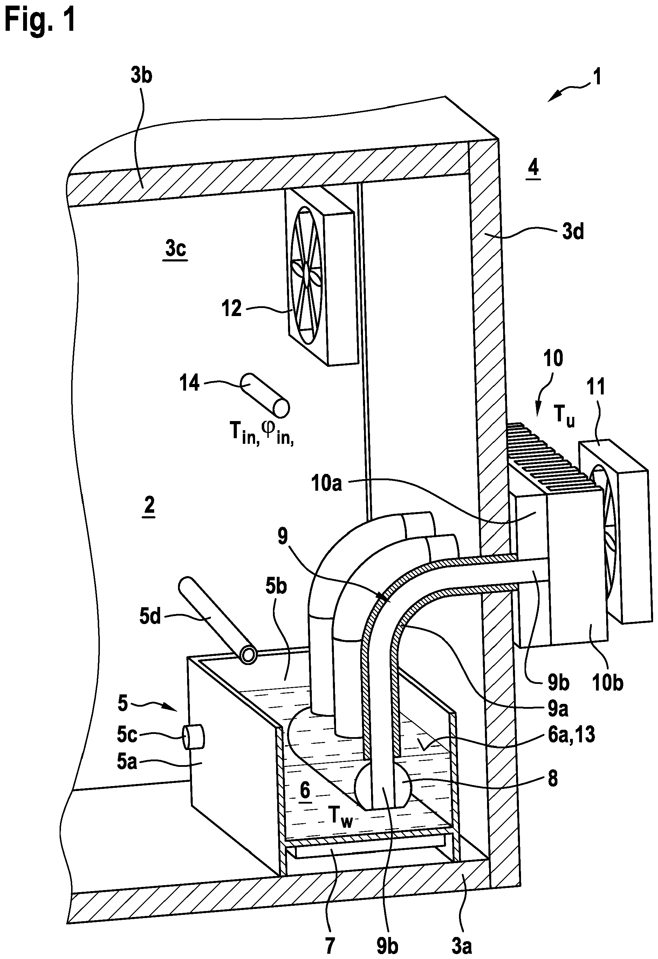

Figur 1 zeigt eine geschnittene, perspektivische Ansicht eines Inkubators mit geregelter Befeuchtung und Temperierung eines Gasgemisches in einem Innenraum (2) den ein Gehäuse (3) umgibt, das einen Boden (3a), eine Decke (3b), zwei Seitenwände (3c), eine Rückwand (3d) sowie eine der Rückwand (3d) gegenüberliegende verschließbare Öffnung, insbesondere in Form einer Tür, aufweist.

Der Innenraum (2) wird durch das Gehäuse (3) gegenüber der Umgebung (4) im technischen Sinne abgedichtet, sodass ein Gasgemisch im Innenraum (2) nicht nur eine abweichende Zusammensetzung gegenüber dem Gasgemisch in der Umgebung, sondern auch abweichende Temperatur und Feuchtigkeit aufweisen kann. Für Kultivierungen werden Temperaturen in dem Innenraum (2) in einem Bereich von etwa 32-37 Grad Celsius sowie Feuchtigkeitswerte im Bereich zwischen 70-90% benötigt.

Um den Wärmedurchgang durch die Wände des Gehäuses zu reduzieren sind die Wände als dreischichtige Verbundkonstruktion in Sandwichbauweise ausgeführt. Die beiden tragenden Deckhäute bestehen aus Edelstahl. Dazwischen befindet sich ein Stützkern aus wärmedämmendem Material, insbesondere PU-Schaum.

Zur Temperierung des Gasgemisches im Innenraum (2) auf eine Innenraumtemperatur Tᵢₙ verfügt der Inkubator (1) über eine Inkubatorheizung, die der Übersichtlichkeit halber in der Figur 1 nicht dargestellt ist. Die Inkubatorheizung kann unmittelbar in die das Gehäuse (3) begrenzenden Platten integriert werden. Hierdurch wird eine gleichmäßige Erwärmung des Innenraums gewährleistet. Darüber hinaus ist es zweckmäßig, auch die Tür des Gehäuses (3) mit einem Heizelement zu versehen, sodass Kondensation von Feuchtigkeit an der vielfach ein Glasfenster aufweisenden Tür durch Ausbildung einer Kältebrücke vermieden wird. Die Inkubatorheizung ist mit einer Regelung verbunden, die die Innenraumtemperatur Tᵢₙ auf einem konstanten Niveau in dem vorgenannten Bereich hält.

Auf dem Boden (3a) des Inkubators (1) ist eine Befeuchtungsvorrichtung (5) zum Verdampfen einer Flüssigkeit angeordnet. Durch Verdampfen der Flüssigkeit wird die Feuchtigkeit ϕᵢₙ des Gasgemisches erhöht. Die Befeuchtungsvorrichtung (5) umfasst einen Behälter (5a) zur Aufnahme der zu verdampfenden Flüssigkeit, eine die Flüssigkeitsoberfläche (6a) freigebende Öffnung (5b) sowie eine an dem Boden des Behälters (5a) angeordnete Heizung (7), insbesondere in Form einer Heizmatte, zur Zuführung thermischer Energie in die Flüssigkeit (6). An einer Seitenwand des Behälters (5a) ist ein Füllstandssensor (5c) angeordnet, der den Flüssigkeitsstand in dem Behälter (5a) überwacht. In den Behälter (5a) mündet eine Flüssigkeitszufuhr (5d), die durch die Seitenwand (3c) hindurch mit einer nicht dargestellten Flüssigkeitsversorgung verbunden ist. Eine Regelung der Füllstandsüberwachung ist derart eingerichtet, dass bei Unterschreiten eines von dem Füllstandssensor (5c) erfassten unteren Grenzwertes für den Füllstand die Flüssigkeit in dem Behälter (5) über die Flüssigkeitszufuhr (5d) automatisch aufgefüllt wird. Hierdurch wird gewährleistet, dass auch bei langandauernden Kultivierungen stets ausreichend Flüssigkeit (6) in dem Behälter (5) enthalten ist, um die Feuchtigkeitsregelung im Innenraum (2) des Inkubators (1) sicherzustellen.

In die Flüssigkeit (6) taucht ein Wärmetauschkörper (8) ein, der mit drei als Wärmerohre ausgestalteten Wärmeleitern (9) wärmeleitend verbunden ist. Die wärmeleitende Verbindung wird durch eine unmittelbare Ankopplung der aus gut wärmeleitendem Material bestehenden Wärmeleiter (9) an den Wärmetauschkörper (8) erreicht, indem die Enden (9b) der Wärmeleiter (9) formschlüssig in korrespondierenden Öffnungen des Wärmetauschkörpers (8) greifen. Um den Wärmeübergangswiderstand zu reduzieren kann Wärmeleitpaste oder Wärmeleitklebefolie zwischen den Enden (9b) und den Öffnungen zum Einsatz gelangen. Zudem können die Wärmeleiter (9) angelötet werden.

Die Wärmeleiter erstrecken sich von dem Wärmetauschkörper (8) in Richtung der Rückwand (3d) zu einem außerhalb des Gehäuses (3) angeordneten Kühlkörper (10). Dabei durchsetzen die Wärmeleiter (9) Öffnungen in der Rückwand (3d) des Gehäuses. Jeder der drei Wärmeleiter (9) weist eine Wärmedämmung (9a) auf, die den Wärmeleiter (9) auch in der Durchtrittsöffnung der Rückwand (3d) umgibt. Der Kühlkörper (10) umfasst eine massive Grundplatte (10a), die an der Rückwand (3d) verschraubt ist. Die Grundplatte (10a) umfasst Öffnungen, in die die nicht wärmegedämmten Enden (9b) der Wärmeleiter (9) formschlüssig eingreifen. Um den Wärmeübergangswiderstand zu reduzieren kann Wärmeleitpaste oder Wärmeleitklebefolie zwischen den Enden (9b) und den Öffnungen zum Einsatz gelangen. Zudem können die Wärmeleiter (9) angelötet werden. Von der Grundplatte (10a) erstrecken sich rechtwinklig zur Rückwand (3d) lamellenartige Kühlbleche (10b) zur Vergrößerung der Oberfläche des Kühlkörpers (10).

An den vertikalen schmalen Seitenflächen der Grundplatte (10a) sind die Schenkel eines u-förmigen Abdeckblechs angeschraubt, das der Übersichtlichkeit halber nicht dargestellt ist. Der die Schenkel verbindende Steg des u-förmigen Abdeckblechs dient der Halterung eines Axiallüfters (11), dessen Drehachse senkrecht auf der Rückwand (3d) steht.

Nahe der Decke (3b) ist in dem Innenraum (2) des Inkubators (1) ein weiterer Lüfter (12) angeordnet, der für eine ausreichende Durchmischung des Gasgemisches in dem Innenraum (2) sorgt, so dass die Temperatur und Feuchtigkeit in dem gesamten Innenraum homogen ist.

Nachfolgend wird das Verfahren zur geregelten Befeuchtung und Temperierung des Gasgemisches in dem Innenraum (2) näher erläutert:
Für die Durchführung eines Kultivierungsprozesses wird das Gasgemisch in dem Innenraum auf eine typische Innenraumtemperatur im Bereich zwischen 32-37 Grad Celsius, die sogenannte Kultivierungstemperatur, temperiert und auf dieser Temperatur im Wesentlichen konstant gehalten. Die Umgebungstemperatur im Labor beträgt beispielsweise zwischen 20 bis 25 Grad Celsius. Bedingt durch die Temperaturdifferenz zwischen der Innenraumtemperatur und der Umgebungstemperatur wird die Flüssigkeit (6) in dem Behälter (5) durch die kontinuierliche Übertragung von Wärme mittels der Wärmeleiter (9) zu dem außerhalb des Gehäuses angeordneten Kühlkörper (10) gekühlt, sodass die Flüssigkeit (6) ohne Wirkung der Heizung (7) eine niedrigere Temperatur als die Innenraumtemperatur Tᵢₙ des Gasgemisches aufweist. Ein Verdampfen von Flüssigkeit aus dem Behälter (5a) wird aufgrund der niedrigeren Temperatur vermieden und an der Flüssigkeitsoberfläche (6a) kondensiert die Feuchtigkeit des Gasgemisches direkt in den Behälter (5a). Hierdurch wird die relative Feuchtigkeit des Gasgemisches in dem Innenraum reduziert. Die kontinuierliche Übertragung von Wärme von der zugleich die Kondensationsoberfläche (13) bildenden Flüssigkeitsoberfläche (6a) zu dem außerhalb des Gehäuses angeordneten Kühlkörper (10) erfolgt über den unterhalb der Kondensationsoberfläche/Flüssigkeitsoberfläche (6a) angeordneten Wärmetauschkörper (8), der wärmeleitend mit den Wärmeleitern (9) verbunden ist.

Zum Erhöhen der Feuchtigkeit des Gasgemisches in dem Innenraum (2) wird mittels der Heizung (7) die Flüssigkeit (6) in dem Behälter soweit erwärmt, dass Flüssigkeit verdampft. Die Verdampfungsleistung wird mithilfe der leistungssteuerbaren Heizung (7) geregelt. Die Regelung erfolgt derart, dass keine Feuchtigkeit an der Flüssigkeitsoberfläche (6a) kondensiert und so viel Flüssigkeit aus dem Behälter (5a) verdampft, bis der Sollwert der Feuchtigkeit ϕₛₒₗₗ erreicht ist, wenn der gemessene Istwert der Feuchtigkeit ϕᵢₙ kleiner als der Sollwert ϕₛₒₗₗ ist. Ist indes der gemessene Istwert ϕᵢₙ größer als der Sollwert ϕₛₒₗₗ wird zur Reduktion solange Feuchtigkeit an derselben Flüssigkeitsoberfläche (6a) kondensiert, bis der Sollwert ϕₛₒₗₗ erreicht ist. Dabei erfolgt die Messung des Istwertes der Feuchtigkeit ϕᵢₙ mittels des in dem Innenraum (2) angeordneten Feuchtigkeitssensors (14).

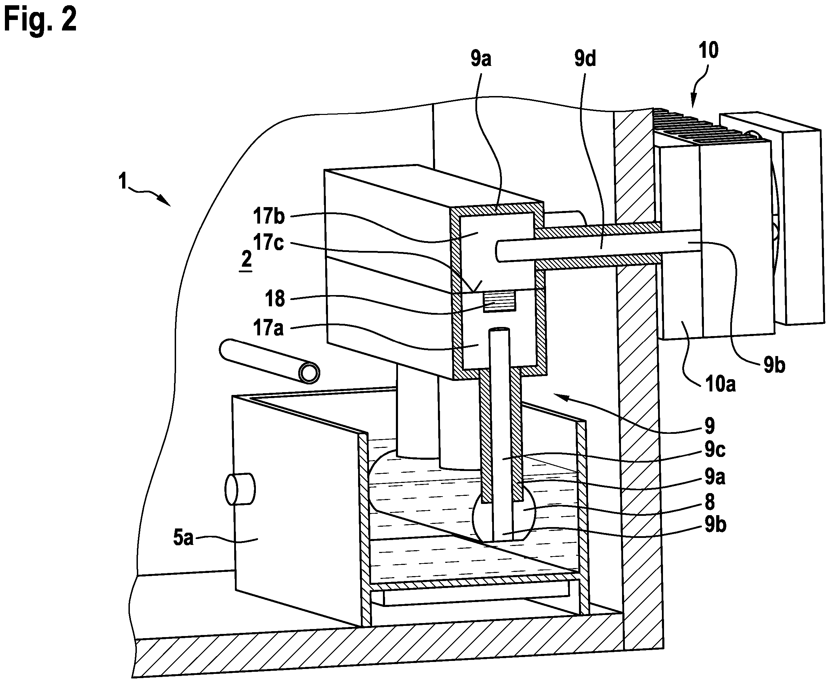

Das Ausführungsbeispiel nach Figur 2 stimmt weitgehend mit dem Ausführungsbeispiel nach Figur 1 überein. Insoweit werden übereinstimmende Bezugszeichen verwendet und es wird auf die Erläuterungen zu Figur 1 verwiesen. Einen Unterschied zu dem Ausführungsbeispiel nach Figur 1 besteht jedoch darin, dass der im Innenraum (2) verlaufende Abschnitt jedes Wärmeleiters (9) aus zwei Abschnitten (9c, 9d) besteht, die wärmeleitend mit Hilfe eines Verbindungsbauteils (17) lösbar miteinander verbunden sind. Das Verbindungsbauteil (17) umfasst einen ersten quaderförmigen Bestandteil (17a) und einen zweiten quaderförmigen Bestandteil (17b). Der erste und zweite Bestandteil (17a, 17b) liegen an einer Grenzfläche (17c) bündig aneinander an und bestehen aus einem thermisch gut leitenden ferromagnetischen Material. In der Grenzfläche (17c) ist in dem ersten Bestandteil (17a) mindestens ein Permanentmagnet (18) angeordnet, der den zweiten Bestandteil (17b) anzieht.

Der erste Abschnitt (9c), jeder der drei Wärmeleiter (9), erstreckt sich geradlinig von dem Wärmetauschkörper (8) bis zu dem ersten Bestandteil (17a) des Verbindungsbauteils (17). Der zweite Abschnitt (9d) jedes Wärmeleiters (9) erstreckt sich von dem zweiten Bestandteil (17b) des Verbindungsbauteils (17) zu der Grundplatte (10a) des Kühlkörpers (10). Die Enden des ersten Abschnitts (9c) jedes Wärmeleiters (9) greifen in entsprechende Öffnungen des ersten Bestandteils (17a) des Verbindungsbauteils (17) sowie des Wärmetauschkörpers(8). Die Enden des zweiten Abschnitts (9d) jedes Wärmeleiters, greifen in entsprechende Öffnungen des zweiten Bestandteils (17b) des Verbindungsbauteils (17) sowie der Grundplatte (10a) des Kühlkörpers (10). Weiter ist erkennbar, dass die Wärmedämmung (9a) auch die nach außen weisenden Flächen des ersten und zweiten Bestandteils (17a, 17b) des Verbindungsbauteils vollständig umgibt, um Kondensation an dem Verbindungsbauteil (17) zu unterbinden.

Zum Reinigen und Entfernen des Behälters (5a) der Befeuchtungsvorrichtung lässt sich die Magnetverbindung entlang der Grenzfläche (17c) unproblematisch lösen, sodass der Wärmetauschkörper (8) zusammen mit den daran angeordneten Abschnitten (9c) der Wärmeleiter (9) sowie dem ersten Bestandteil (17a) des Verbindungsbauteils entfernt werden kann. Anschließend lässt sich der Behälter (5a) problemlos unter dem in dem Gehäuse (3) verbleibenden zweiten Bestandteil (17b) des Verbindungsbauteils und den Abschnitten (9d) der Wärmeleiter (9) herausziehen.

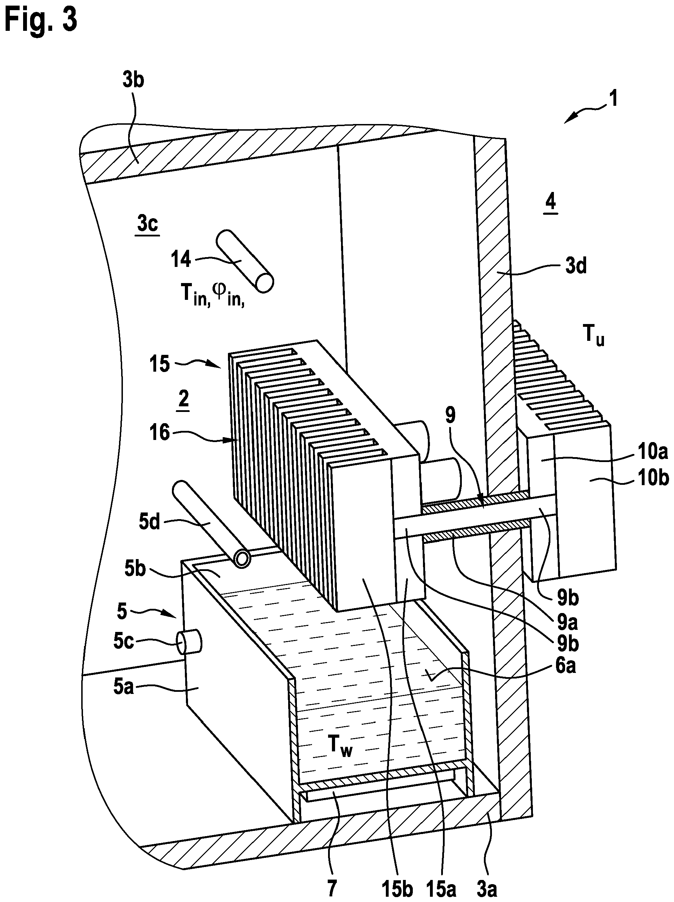

Das Ausführungsbeispiel des Inkubators nach Figur 3 stimmt teilweise mit dem Ausführungsbeispiel nach Figur 1 überein. Insoweit werden übereinstimmende Bezugszeichen verwendet und es wird auf die Erläuterungen zu Figur 1 verwiesen. Unterschiede ergeben sich insoweit als die Feuchtigkeit des Gasgemisches nicht an der Flüssigkeitsoberfläche (6a), sondern an der Oberfläche eines Wärmetauschkörpers (15) kondensiert, der im Innenraum (2) des Inkubators (1) oberhalb der Öffnung (5b) des Behälters (5a) der Befeuchtungsvorrichtung (5) angeordnet ist.

Der Wärmetauschkörper (15) ist im Wesentlichen übereinstimmend wie der außerhalb des Gehäuses angeordnete Kühlkörper (10) aufgebaut. Er umfasst eine massive Grundplatte (15a), in die die nicht wärmegedämmten Enden (9b) der drei Wärmeleiter (9) formschlüssig eingreifen. Um den Wärmeübergangswiderstand zu reduzieren kann Wärmeleitpaste oder Wärmeleitklebefolie zwischen den Enden (9b) und den Öffnungen zum Einsatz gelangen. Zudem können die Wärmeleiter angelötet werden. Von der Grundplatte (15a) erstrecken sich auf der gegenüberliegenden Seite lamellenartige Bleche (15b), die die Fläche für den Wärmeübergang vergrößern. Die gesamte, mit dem Gasgemisch in Kontakt gelangende Oberfläche des Wärmeaufnahmekörpers, d.h. die Oberfläche der Bleche (15b) sowie der Grundplatte (15a) bilden die Kondensationsoberfläche. Die an dieser Kondensationsoberfläche kondensierende Feuchtigkeit tropft aufgrund der Anordnung oberhalb der Öffnung (5b) des Behälters (5a) unmittelbar in die Flüssigkeit (6), sodass Kontaminationen durch kondensierende Flüssigkeit ausgeschlossen sind.

Abweichend zu dem Ausführungsbeispiel nach Figur 1 sind der Wärmeaufnahmekörper (15) und der Kühlkörper (10) über gerade Wärmeleiter (9) auf deutlich kürzerem Weg durch die Rückwand (3d) hindurch miteinander verbunden.

Hinsichtlich der Regelung der Verdampfungsleitung mittels der Heizung (7) ergibt sich insoweit ein Unterschied zu dem Ausführungsbeispiel nach Figur 1, dass aufgrund der kontinuierlichen Kondensation an dem Wärmetauschkörper (15) die Verdampfung auch bei einer angestrebten Reduktion der Feuchtigkeit des Gasgemisches in dem Innenraum nicht zwingend vollständig unterbunden werden muss. Hierdurch lässt sich der zeitliche Verlauf der Feuchtigkeitsreduktion durch gezielte Steuerung der Verdampfungsleistung bei gleichzeitiger Kondensation an dem Wärmetauschkörper (15) steuern. Im Einzelnen wird die Verdampfungsleistung bei einem Inkubator (1) nach Figur 2 derart geregelt, dass so viel Flüssigkeit aus dem Behälter (5a) im Verhältnis zu der kontinuierlich an der Kondensationsoberfläche (16) des Wärmeaustauschkörpers kondensierenden Feuchtigkeit verdampft, bis der Sollwert ϕₛₒₗₗ der Feuchtigkeit erreicht ist, wenn der gemessene Istwert kleiner als der Sollwert ist. Um einen raschen Ausgleich einer Regelabweichung zu erreichen, wird die Befeuchtungsvorrichtung (5) vorzugsweise mit maximaler Heizleistung der Heizung (7) betrieben.

Ist der Istwert ϕᵢₙ der Feuchtigkeit indes größer als der Sollwert wird vorzugsweise keine oder so wenig Flüssigkeit im Verhältnis zu der kontinuierlich an der Kondensationsoberfläche (16) kondensierenden Feuchtigkeit verdampft, bis der Sollwert der Feuchtigkeit erreicht ist. Eine Aufrechterhaltung einer geringen Verdampfung kann im Rahmen der Regelung sinnvoll sein, sofern eine zeitlich langsame Absenkung der Feuchtigkeit gewünscht wird.

| Nr. | **Bezeichnung** |
|---|---|
| 1. | Inkubator |
| 2. | Innenraum |
| 3. | Gehäuse |
| 3a. | Boden |
| 3b. | Decke |
| 3c. | Seitenwand |
| 3d. | Rückwand |
| 4. | Umgebung |
| 5. | Befeuchtungsvorrichtung |
| 5a. | Behälter |
| 5b. | Öffnung |
| 5c. | Füllstandssensor |
| 5d. | Flüssigkeitszufuhr |
| 6. | Flüssigkeit |
| 6a. | Flüssigkeitsoberfläche |
| 7. | Heizung |
| 8. | Wärmetauschkörper |
| 9. | Wärmeleiter |
| 9a. | Wärmedämmung |
| 9b. | Enden Wärmeleiter |
| 9c. | Abschnitte |
| 9d. | Abschnitte |
| 10. | Kühlkörper |
| 10a. | Grundplatte |
| 10b. | Kühlblech |
| 11./12. | Lüfter |
| 13. | Kondensationsoberfläche |
| 14. | Feuchtigkeitssensor |
| 15. | Wärmetauschkörper |
| 15a. | Grundplatte |
| 15b. | Bleche |
| 16. | Kondensationsoberfläche |
| 17. | Verbindungsbauteil |
| 17a. | erster Bestandteil |
| 17b. | zweiter Bestandteil |
| 17c. | Grenzfläche |
| 18. | Permanentmagnet |

## Patentansprüche

1. Verfahren zur geregelten Befeuchtung und Temperierung eines Gasgemisches in einem gegen die Umgebung mittels eines Gehäuses (3) abgedichteten Innenraum (2) eines Inkubators (1), umfassend die Schritte
- Temperieren des Gasgemisches auf eine Innenraumtemperatur, die höher als eine außerhalb des Inkubators herrschende Umgebungstemperatur ist,
- Verdampfen einer Flüssigkeit in dem Innenraum (2) mittels einer Befeuchtungsvorrichtung (5), deren Verdampfungsleistung regelbar ist und/oder Kondensieren der Feuchtigkeit des Gasgemisches an mindestens einer Kondensationsoberfläche (13, 16) in dem Innenraum (2),
- kontinuierliches Übertragen von Wärme mit Hilfe mindestens eines Wärmeleiters (9) von der mindestens einen Kondensations-Oberfläche (13, 16) zu einem außerhalb des Gehäuses(3) angeordneten Kühlkörper (10), wobei der Wärmeübergang von dem Kühlkörper (10) zu der Umgebungsluft durch Verändern der Strömung der Umgebungsluft entlang wärmeabgebender Oberflächen des Kühlkörpers (10) mittels mindestens eines Lüfters (11) veränderbar ist,
- Messen eines Istwertes der Feuchtigkeit des Gasgemisches in dem Innenraum (2),
- Vergleichen des Istwertes mit einem Sollwert für die Feuchtigkeit,
- Regelung der Verdampfungsleistung wenn eine Abweichung des Istwertes von dem Sollwert vorliegt.

2. Verfahren nach Anspruch 1, **dadurch gekennzeichnet, dass** das Verdampfen der Flüssigkeit (6) in einem eine Öffnung (5b) aufweisenden beheizbaren Behälter (5a) erfolgt, wobei die Öffnung (5b) die Flüssigkeitsoberfläche (6a) freigibt und die Verdampfungsleistung mit der Heizung (7) geregelt wird.

3. Verfahren nach Anspruch 2, **dadurch gekennzeichnet, dass** die Feuchtigkeit des Gasgemisches an der Flüssigkeitsoberfläche (6a) kondensiert, die zugleich die Kondensations-Oberfläche (13) bildet.

4. Verfahren nach Anspruch 3, **dadurch gekennzeichnet, dass** der mindestens ein Wärmeleiter (9) mit einem Wärmetauschkörper (8) wärmeleitend verbunden ist und der Wärmetauschkörper (8) zumindest teilweise in die Flüssigkeit (6) eintaucht.

5. Verfahren nach Anspruch 3 oder 4, **dadurch gekennzeichnet, dass** die Verdampfungsleistung derart geregelt wird, dass
- keine Feuchtigkeit an der Flüssigkeitsoberfläche (6a) kondensiert und so viel Flüssigkeit (6) verdampft, bis der Sollwert der Feuchtigkeit erreicht ist, wenn der gemessene Istwert kleiner als der Sollwert ist, oder
- Feuchtigkeit an der Flüssigkeitsoberfläche (6a) kondensiert und keine Flüssigkeit verdampft, bis der Sollwert der Feuchtigkeit erreicht ist, wenn der gemessene Istwert größer als der Sollwert ist.

6. Verfahren nach Anspruch 1 oder 2, **dadurch gekennzeichnet, dass** die Feuchtigkeit an der Kondensationsoberfläche (16) eines Wärmetauschkörpers (15) kondensiert, der in dem Innenraum (2) des Inkubators (1) angeordnet ist.

7. Verfahren nach Anspruch 6, **dadurch gekennzeichnet, dass** die Verdampfungsleistung derart geregelt wird, dass
- so viel Flüssigkeit im Verhältnis zu der kontinuierlich an der Kondensationsoberfläche (16) kondensierenden Feuchtigkeit verdampft, bis der Sollwert der Feuchtigkeit erreicht ist, wenn der gemessene Istwert kleiner als der Sollwert ist,
- keine oder so wenig Flüssigkeit im Verhältnis zu der kontinuierlich an der Kondensationsoberfläche (16) kondensierenden Feuchtigkeit verdampft, bis der Sollwert der Feuchtigkeit erreicht ist, wenn der gemessene Istwert größer als der Sollwert ist.

8. Verfahren nach einem der Ansprüche 1 bis 7, **dadurch gekennzeichnet, dass** das Gasgemisch im Innenraum kontinuierlich umgewälzt wird.

9. Verfahren nach einem der Ansprüche 1 bis 8, **dadurch gekennzeichnet, dass** die Innenraumtemperatur auf einen konstanten Wert im Bereich von 32° C - 40 °C geregelt wird.

10. Verfahren nach einem der Ansprüche 1 bis 9, **dadurch gekennzeichnet, dass** die Umgebungstemperatur im Bereich von 20°C - 25°C liegt.

11. Inkubator (1) mit geregelter Befeuchtung und Temperierung eines Gasgemisches in einem abgedichteten Innenraum (2), wobei das Gasgemisch eine Innenraumtemperatur aufweist, die höher als eine außerhalb des Inkubators (1) herrschende Umgebungstemperatur ist, umfassend
- ein Gehäuse (3) mit einer verschließbaren Öffnung, das den Innenraum (2) umgibt,
- eine Befeuchtungsvorrichtung (5) zum Verdampfen einer Flüssigkeit mit regelbarer Verdampfungsleistung eingerichtet zum Erhöhen der Feuchtigkeit des Gasgemisches in dem Innenraum (2),
- eine in dem Innenraum (2) angeordnete Kondensations-Oberfläche (13, 16) zum Reduzieren der Feuchtigkeit des Gasgemisches durch Kondensation,
- einen außerhalb des Gehäuses (3) angeordneten Kühlkörper (10), wobei an dem Kühlkörper (10) mindestens ein in der Drehzahl steuerbarer Lüfter (11) derart angebracht ist, dass er eine Strömung der Umgebungsluft entlang wärmeabgebender Oberflächen des Kühlkörpers (10) erzeugt,
- mindestens einen Wärmeleiter (9) zum Übertragen von Wärme von der Kondensationsoberfläche (13, 16) zu dem Kühlkörper (10),
- einen Feuchtigkeitssensor (14) zum Messen eines Istwertes der Feuchtigkeit des Gasgemisches in dem Innenraum (2) und
- eine Feuchtigkeitsregelung, eingerichtet zum Vergleichen des Istwertes mit einem Sollwert für die Feuchtigkeit sowie zur Regelung der Verdampfungsleistung, wenn eine Abweichung des Istwertes von dem Sollwert vorliegt.

12. Inkubator nach Anspruch 11, **dadurch gekennzeichnet, dass** die Befeuchtungsvorrichtung (5) einen Behälter (5a) zur Aufnahme von zu verdampfender Flüssigkeit (6), mindestens eine die Flüssigkeitsoberfläche (6a) freigebende Öffnung (5b) in dem Behälter (5a) sowie eine Heizung (7) zur Zuführung thermischer Energie in die Flüssigkeit (6) aufweist.

13. Inkubator nach Anspruch 12, **dadurch gekennzeichnet, dass**
- ein Wärmetauschkörper (8) zumindest teilweise in die Flüssigkeit (6) eintaucht,
- der mindestens eine Wärmeleiter (9) mit dem Wärmetauschkörper (8) wärmeleitend verbunden ist und
- die Flüssigkeitsoberfläche (6a) zugleich die Kondensationsoberfläche (13) bildet.

14. Inkubator nach einem der Ansprüche 11 bis 13, **dadurch gekennzeichnet, dass** der im Innenraum (2) befindliche Abschnitt jedes Wärmeleiters (9) aus mehreren, lösbar miteinander verbundenen Bestandteilen besteht.

15. Inkubator nach Anspruch 14, **dadurch gekennzeichnet, dass** ein erster und ein zweiter Bestandteil (9c, 9d) jedes Wärmeleiters (9) durch eine Magnetverbindung (18) lösbar miteinander verbunden sind.

16. Inkubator nach Anspruch 13 und 15, **dadurch gekennzeichnet, dass**
- der erste Bestandteil (9c) jedes Wärmeleiters (9) wärmeleitend mit einem ersten Teil (17a) eines wärmeleitenden Verbindungsbauteils (17) verbunden ist,
- der zweite Bestandteil (9d) jedes Wärmeleiters (9) wärmeleitend mit einem zweiten Teil (17b) des Verbindungsbauteils (17) verbunden ist und
- der erste und zweite Teil (17a, 17b) des Verbindungsbauteils (17) eine Grenzfläche (17c) aufweisen, in der die Magnetverbindung (18) wirksam ist.

17. Inkubator nach Anspruch 12, **dadurch gekennzeichnet, dass** in dem Innenraum (2) ein Wärmetauschkörper (15) oberhalb des Behälters (5a) angeordnet ist, dessen Oberfläche (16) zumindest teilweise die Kondensationsoberfläche bildet.

18. Inkubator nach Anspruch 17, **dadurch gekennzeichnet, dass** der Wärmetauschkörper (15) oberhalb der die Flüssigkeitsoberfläche (6a) freigebenden Öffnung (5b) des Behälters (5a) angeordnet ist.

19. Inkubator nach einem der Ansprüche 11 bis 18, **dadurch gekennzeichnet, dass** jeder Wärmeleiter (9) als Wärmerohr ausgebildet ist.

20. Inkubator nach einem der Ansprüche 11 bis 16, **dadurch gekennzeichnet, dass** an jedem Wärmeleiter (9) eine Wärmedämmung (9a) angeordnet ist.

21. Inkubator nach einem der Ansprüche 11 bis 20, **dadurch gekennzeichnet, dass** die Feuchtigkeitsregelung, weiter derart eingerichet ist, dass jeder Lüfter (11) beim Erhöhen der Feuchtigkeit abgeschaltet oder mit niedrigerer Drehzahl als beim Reduzieren der Feuchtigkeit betrieben wird.

22. Inkubator nach einem der Ansprüche 11 bis 21, **dadurch gekennzeichnet, dass** im Innenraum (2) mindestens ein Lüfter (12) zur Umwälzung des Gasgemisches in dem Innenraum (2) angeordnet ist.

23. Inkubator nach einem der Ansprüche 12 bis 22, **dadurch gekennzeichnet, dass**
- an dem Behälter (5a) ein Füllstandssensor (5c) für die Erfassung des Füllstandes der Flüssigkeit (6) angeordnet ist,
- in dem Behälter (5a) eine Flüssigkeitszufuhr (5d) mündet, die mit einer Flüssigkeitsversorgung verbunden ist und
- eine Steuerung derart eingerichtet ist, dass bei Unterschreiten eines von dem Füllstandssensor (5c) erfassten unteren Grenzwertes für den Füllstand, die Flüssigkeit in dem Behälter (5a) über die Flüssigkeitszufuhr (5d) aufgefüllt wird.

## Claims

1. A method for regulated humidification and temperature control of a gas mixture in an inner chamber (2) of an incubator (1), which inner chamber is sealed off from the surroundings by a housing (3), comprising the steps of
- controlling the temperature of the gas mixture to an inner chamber temperature which is higher than an ambient temperature prevailing outside the incubator,
- evaporating a liquid in the inner chamber (2) by means of a humidification device (5) whose evaporative capacity can be regulated and/or condensing the moisture of the gas mixture on at least one condensation surface (13, 16) in the inner chamber (2),
- continuously transferring heat from the at least one condensation surface (13, 16) to a cooling body (10) arranged outside the housing (3) with the aid of at least one heat conductor (9), wherein the heat transfer from the cooling body (10) to the ambient air can be varied by varying the flow of the ambient air along heat-dissipating surfaces of the cooling body (10) by means of at least one fan (11),
- measuring an actual value of the humidity of the gas mixture in the inner chamber (2),
- comparing the actual value with a target value for the humidity,
- regulating the evaporative capacity if there is a deviation of the actual value from the target value.

2. The method according to claim 1, **characterized in that** the evaporation of the liquid (6) is accomplished in a heatable container (5a) which has an opening (5b), wherein the opening (5b) exposes the liquid surface (6a) and the evaporative capacity is regulated with the heater (7).

3. The method according to claim 2, **characterized in that** the moisture of the gas mixture condenses on the liquid surface (6a) which at the same time forms the condensation surface (13).

4. The method according to claim 3, **characterized in that** the at least one heat conductor (9) is connected in a heat-conducting manner to a heat exchange body (8), and the heat exchange body (8) is at least partially submerged in the liquid (6).

5. The method according to claim 3 or 4, **characterized in that** the evaporative capacity is regulated in such a manner that
- no moisture condenses on the liquid surface (6a) and so much liquid (6) evaporates, until the target value of the humidity is reached if the measured actual value is less than the target value, or
- moisture condenses on the liquid surface (6a) and no liquid evaporates until the target value of the humidity is reached if the measured actual value is greater than the target value.

6. The method according to claim 1 or 2, **characterized in that** the moisture condenses on the condensation surface (16) of a heat exchange body (15) arranged in the inner chamber (2) of the incubator (1).

7. The method according to claim 6, **characterized in that** the evaporative capacity is regulated in such a manner that
- so much liquid is evaporated in relation to the moisture condensing continuously on the condensation surface (16), until the target value of the humidity is reached if the measured actual value is less than the target value,
- none or so little liquid is evaporated in relation to the moisture condensing continuously on the condensation surface (16), until the target value of the humidity is reached if the measured actual value is greater than the target value.

8. The method according to one of claims 1 to 7, **characterized in that** the gas mixture is continuously circulated in the inner chamber.

9. The method according to one of claims 1 to 8, **characterized in that** the inner chamber temperature is regulated to a constant value in the range of 32°C - 40°C.

10. The method according to one of claims 1 to 9, **characterized in that** the ambient temperature lies in the range of 20°C - 25°C.

11. An incubator (1) with regulated humidification and temperature control of a gas mixture in a sealed-off inner chamber (2), wherein the gas mixture has an inner chamber temperature which is higher than an ambient temperature prevailing outside the incubator (1), the incubator comprising:
- a housing (3) surrounding the inner chamber (2) and having a closable opening,
- a humidification device (5) to evaporate a liquid with regulable evaporative capacity, adapted to increase the humidity of the gas mixture in the inner chamber (2),
- a condensation surface (13, 16) arranged in the inner chamber (2) to reduce the humidity of the gas mixture by condensation,
- a cooling body (10) arranged outside the housing (3), wherein at least one controllable-speed fan (11) is mounted on the cooling body (10) in such a manner that it generates a flow of ambient air along heat-dissipating surfaces of the cooling body (10),
- at least one heat conductor (9) to transfer heat from the condensation surfaces (13, 16) to the cooling body (10),
- a humidity sensor (14) for measuring an actual value of the humidity of the gas mixture in the inner chamber (2), and
- a humidity regulator adapted to compare the actual value with a target value for the humidity, and for regulating the evaporative capacity if there is a deviation of the actual value from the target value.

12. The incubator according to claim 11, **characterized in that** the humidification device (5) has a container (5a) for holding liquid (6) to be evaporated, at least one opening (5b) exposing the liquid surface (6a) in the container, and a heater (7) for feeding thermal energy into the liquid (6).

13. The incubator according to claim 12, **characterized in that**
- a heat exchange body (8) is at least partially submerged in the liquid (6),
- the at least one heat conductor (9) is connected in a heat-conducting manner to the heat exchange body (8), and
- the liquid surface (6a) at the same time forms the condensation surface (13).

14. The incubator according to one of claims 11 to 13, **characterized in that** the section of each heat conductor (9) located in the inner chamber (2) consists of a plurality of detachably interconnected constituent parts.

15. The incubator according to claim 14, **characterized in that** a first and a second constituent part (9c, 9d) of each heat conductor (9) are detachably interconnected by a magnetic connection (18).

16. The incubator according to claim 13 and 15, **characterized in that**
- the first constituent part (9c) of each heat conductor (9) is connected in a heat-conducting manner to a first part (17a) of a heat-conducting connecting component (17),
- the second constituent part (9d) of each heat conductor (9) is connected in a heat-conducting manner to a second part (17b) of the connecting component (17), and
- the first and second part (17a, 17b) of the connecting component (17) have a boundary surface (17c) at which the magnetic connection (18) is active.

17. The incubator according to claim 12, **characterized in that** a heat exchange body (15) is arranged in the inner chamber (2) above the container (5a), the surface (16) of which at least partially forms the condensation surface.

18. The incubator according to claim 17, **characterized in that** the heat exchange body (15) is arranged above the opening (5b) of the container (5a) which exposes the liquid surface (6a).

19. The incubator according to one of claims 11 to 18, **characterized in that** each heat conductor (9) is configured as a heat pipe.

20. The incubator according to one of claims 11 to 16, **characterized in that** a heat insulation (9a) is arranged on each heat conductor (9).

21. The incubator according to one of claims 11 to 20, **characterized in that** the humidity regulation is further configured in such a manner that when the humidity increases, each fan (11) is switched off or is operated at a lower rotational speed than when the humidity is reduced.

22. The incubator according to one of claims 11 to 21, **characterized in that** at least one fan (12) is arranged in the inner chamber (2) to circulate the gas mixture in the inner chamber (2).

23. The incubator according to one of claims 12 to 22, **characterized in that**
- a fill level sensor (5c) is arranged on the container (5a) for detecting the fill level of the liquid (6),
- a liquid feed (5d) which is connected to a liquid supply opens into the container (5a), and
- a control is adapted in such a manner that when the fill level drops below a lower limit value detected by the fill level sensor (5c), the liquid in the container (5a) is replenished via the liquid feed (5d).

## Revendications

1. Procédé, destiné à l'humidification régulée et à la mise en température d'un mélange gazeux dans un espace intérieur (2) d'un incubateur (1) étanchéifié par rapport à l'environnement à l'aide d'un boîtier (3), comprenant les étapes consistant à
- tempérer le mélange gazeux à une température d'espace intérieur qui est supérieure à une température ambiante régnant à l'extérieur de l'incubateur,
- faire évaporer un liquide dans l'espace intérieur (2) à l'aide d'un dispositif humidificateur (5), dont la puissance d'évaporation est réglable et/ou
faire condenser l'humidité du mélange gazeux sur au moins une surface de condensation (13, 16) dans l'espace intérieur (2),
- transférer en continu de la chaleur à l'aide d'au moins un conducteur thermique (9) de l'au moins une surface de condensation (13, 16) vers un dissipateur thermique (10) placé à l'extérieur du boîtier (3), le transfert thermique du dissipateur thermique (10) vers l'air ambiant étant variable par variation de la circulation de l'air ambiant le long de surfaces dispensant de la chaleur du dissipateur thermique (10) à l'aide d'au moins un ventilateur (11),
- mesurer une valeur réelle de l'humidité du mélange gazeux dans l'espace intérieur (2),
- comparer la valeur réelle avec une valeur de consigne pour l'humidité,
- régler la puissance d'évaporation en présence d'un écart entre la valeur réelle et la valeur de consigne.

2. Procédé selon la revendication 1, **caractérisé en ce que** l'évaporation du liquide (6) a lieu dans un réservoir (5a) susceptible d'être chauffé, comportant un orifice (5b), l'orifice (5b) libérant la surface du liquide (6a) et la puissance d'évaporation étant réglée par le chauffage (7).

3. Procédé selon la revendication 2, **caractérisé en ce que** l'humidité du mélange gazeux se condense sur la surface du liquide (6a) qui forme également la surface de condensation (13).

4. Procédé selon la revendication 3, **caractérisé en ce que** l'au moins un conducteur thermique (9) est relié de manière à conduire la chaleur avec un corps échangeur thermique (8) et le corps échangeur thermique (8) plonge au moins en partie dans le liquide (6).

5. Procédé selon la revendication 3 ou 4, **caractérisé en ce qu'**on règle la puissance d'évaporation de telle sorte
- qu'aucune humidité ne se condense sur la surface du liquide (6a) et qu'une quantité de liquide (6) telle s'évapore jusqu'à ce que la valeur de consigne pour l'humidité soit atteinte, si la valeur réelle mesurée est inférieure à la valeur de consigne, ou
- que l'humidité sur la surface du liquide (6a) se condense et qu'aucun liquide ne s'évapore jusqu'à ce que la valeur de consigne pour l'humidité soit atteinte, si la valeur réelle mesurée est supérieure à la valeur de consigne.

6. Procédé selon la revendication 1 ou 2, **caractérisé en ce que** l'humidité se condense sur la surface de condensation (16) d'un corps échangeur thermique (15) qui est placé dans l'espace intérieur (2) de l'incubateur (1).

7. Procédé selon la revendication 6, **caractérisé en ce qu'**on règle la puissance d'évaporation de telle sorte
- qu'une quantité de liquide telle s'évapore en rapport à l'humidité condensée continuellement sur la surface de condensation (16) jusqu'à ce que la valeur de consigne de l'humidité soit atteinte, si la valeur réelle mesurée est inférieure à la valeur de consigne,
- qu'aucun ou si peu de liquide s'évapore en rapport à l'humidité condensée continuellement sur la surface de condensation (16) jusqu'à ce que la valeur de consigne de l'humidité soit atteinte, si la valeur réelle mesurée est supérieure à la valeur de consigne.

8. Procédé selon l'une quelconque des revendications 1 à 7, **caractérisé en ce qu'**on brasse continuellement le mélange gazeux dans l'espace intérieur.

9. Procédé selon l'une quelconque des revendications 1 à 8, **caractérisé en ce qu'**on règle la température d'espace intérieur à une valeur constante de l'ordre de 32° C à 40 °C.

10. Procédé selon l'une quelconque des revendications 1 à 9, **caractérisé en ce que** la température ambiante se situe dans l'ordre de 20°C à 25°C.

11. Incubateur (1) pourvu d'une humidification régulée et d'une mise en température d'un mélange gazeux dans un espace intérieur (2) étanchéifié, le mélange gazeux présentant une température d'espace intérieur qui est supérieure à une température ambiante régnant à l'extérieur de l'incubateur (1), comprenant
- un boîtier (3) pourvu d'un orifice susceptible d'être fermé, qui entoure l'espace intérieur (2),
- un dispositif humidificateur (5), destiné à faire évaporer un liquide à une puissance d'évaporation réglable, aménagé pour augmenter l'humidité du mélange gazeux dans l'espace intérieur (2),
- une surface de condensation (13, 16) placée dans l'espace intérieur (2), destinée à réduire par condensation l'humidité du mélange gazeux,
- un dissipateur thermique (10), placé à l'extérieur du boîtier (3), un ventilateur (11) dont la vitesse de rotation est susceptible d'être commandée étant monté sur le dissipateur thermique (10) de telle sorte qu'il génère une circulation de l'air ambiant le long de surfaces dispensatrices de chaleur du dissipateur thermique (10),
- au moins un conducteur thermique (9), destiné à transférer de la chaleur de la surface de condensation (13, 16) vers le dissipateur thermique (10),
- un capteur d'humidité (14), destiné à mesurer une valeur réelle de l'humidité du mélange gazeux dans l'espace intérieur (2) et
- une régulation d'humidité, aménagée pour comparer la valeur réelle avec une valeur de consigne pour l'humidité ainsi que pour réguler la puissance d'évaporation, en présence d'un écart entre la valeur réelle et la valeur de consigne.

12. Incubateur selon la revendication 11, **caractérisé en ce que** le dispositif humidificateur (5) comporte un réservoir (5a), destiné à recevoir du liquide (6) qui doit s'évaporer, au moins un orifice (5b) libérant la surface du liquide (6a) dans le réservoir (5a), ainsi qu'un chauffage (7) destiné à amener de l'énergie thermique dans le liquide (6).

13. Incubateur selon la revendication 12, **caractérisé**
- **en ce qu'**un corps échangeur thermique (8) plonge au moins partiellement dans le liquide (6),
- **en ce que** l'au moins un conducteur thermique (9) est relié de manière à conduire la chaleur avec le corps échangeur thermique (8) et
- **en ce que** la surface du liquide (6a) forme simultanément la surface de condensation (13).

14. Incubateur selon l'une quelconque des revendications 11 à 13, **caractérisé en ce que** le tronçon de chaque conducteur thermique (9) qui se trouve dans l'espace intérieur (2) est constitué de plusieurs éléments, reliés les uns aux autres de manière amovible.

15. Incubateur selon la revendication 14, **caractérisé en ce qu'**un premier et un deuxième éléments (9c, 9d) de chaque conducteur thermique (9) sont reliés les uns aux autres de manière amovible par une liaison aimantée (18).

16. Incubateur selon la revendication 13 et 15, **caractérisé en ce que**
- le premier élément (9c) de chaque conducteur thermique (9) est relié de manière à conduire la chaleur avec une première partie (17a) d'un élément de liaison (17) conducteur de chaleur,
- le deuxième élément (9d) de chaque conducteur thermique (9) est relié de manière à conduire la chaleur avec une deuxième partie (17b) de l'élément de liaison (17) et
- les première et deuxième parties (17a, 17b) de l'élément de liaison (17) comportent une surface limite (17c) dans laquelle la liaison aimantée (18) est active.

17. Incubateur selon la revendication 12, **caractérisé en ce que** dans l'espace intérieur (2), un corps échangeur thermique (15) dont la surface (16) forme au moins partiellement la surface de condensation est placé au-dessus du réservoir (5a).

18. Incubateur selon la revendication 17, **caractérisé en ce que** le corps échangeur thermique (15) est placé au-dessus de l'orifice (5b) du réservoir (5a) qui libère la surface du liquide (6a).

19. Incubateur selon l'une quelconque des revendications 11 à 18, **caractérisé en ce que** chaque conducteur thermique (9) est conçu sous la forme d'un caloduc.

20. Incubateur selon l'une quelconque des revendications 11 à 16, **caractérisé en ce que** sur chaque conducteur thermique (9) est placée une isolation thermique (9a).

21. Incubateur selon l'une quelconque des revendication 11 à 20, **caractérisé en ce que** la régulation d'humidité, est aménagée par ailleurs de telle sorte que lors de l'augmentation de l'humidité, chaque ventilateur (11) se coupe ou fonctionne avec une vitesse de rotation plus faible que lors de la réduction de l'humidité.

22. Incubateur selon l'une quelconque des revendications 11 à 21, **caractérisé en ce que** dans l'espace intérieur (2) est placé au moins un ventilateur (12), destiné à brasser le mélange gazeux dans l'espace intérieur (2).

23. Incubateur selon l'une quelconque des revendications 12 à 22, **caractérisé en ce que**
- sur le réservoir (5a) est placé un capteur de niveau (5c) destiné à détecter le niveau de remplissage du liquide (6),
- dans le réservoir (5a) débouche une amenée de liquide (5d) qui est reliée avec une alimentation de liquide et
- un système de commande est aménagé de telle sorte que lors de la non-atteinte d'une valeur limite inférieure détectée par le capteur de niveau (5c) pour le niveau de remplissage, on remplit le réservoir (5a) de liquide via l'amenée de liquide (5d) .
